(19) Europäisches Patentamt
European Patent Office
Office européen des brevets

(11) **EP 4 552 734 A1**

(12) **EUROPEAN PATENT APPLICATION**

(43) Date of publication:
14.05.2025  Bulletin 2025/20

(21) Application number: 23306919.4

(22) Date of filing: 07.11.2023

(51) International Patent Classification (IPC):
*B01J 13/16* (2006.01)    *A61K 8/11* (2006.01)
*A61Q 5/00* (2006.01)    *A61Q 19/00* (2006.01)
*A62D 1/00* (2006.01)    *C11D 3/50* (2006.01)

(52) Cooperative Patent Classification (CPC):
**B01J 13/16; A61K 8/11; A61K 8/37; A61K 8/8152; A61K 8/87; A61K 8/922; A61Q 5/02; A61Q 5/12; A61Q 19/00; A61Q 19/10; C11D 3/37; C11D 3/505; C11D 17/0039; A61K 2800/412**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Calyxia**
**94380 Bonneuil-sur-Marne (FR)**

(72) Inventors:
• **BROSSE, Alexandre**
  **51110 Isles-sur-Suippe (FR)**
• **BLANGEARD, Quentin**
  **94210 Saint-Maur-des-Fossés (FR)**
• **OUHENIA, Karima**
  **93330 Neuilly sur Marne (FR)**
• **SADAOUI ROUSSELLE, Alicia**
  **91160 Saulx les Chartreux (FR)**

(74) Representative: **Lavoix**
**2, place d'Estienne d'Orves**
**75441 Paris Cedex 09 (FR)**

(54) **MICROCAPSULES CONTAINING VOLATILE INGREDIENTS**

(57)    The present invention relates to a biodegradable solid cross-linked microcapsule encapsulating at least one volatile ingredient or a preparation comprising a volatile ingredient, said microcapsule having a mean diameter from 0.5 $\mu$m to 100 $\mu$m, said microcapsule comprising:
- a core consisting of a composition C1 comprising at least one volatile ingredient or a preparation comprising a volatile ingredient, and from 1% to 20% by weight of a viscosifying agent relative to the total weight of said composition, and

- a solid cross-linked polymer envelope at least partially, preferably totally, encapsulating the core at its periphery,

said solid cross-linked polymer envelope being made of a cross-linked polymer, said polymer being a polymer obtained by polymerizing one or several oligomer(s) or monomer(s),
and wherein the average thickness of said solid cross-linked polymer envelope is from 5% to 30% of the average diameter of said solid cross-linked microcapsule.

**Description**

The present invention concerns microcapsules containing at least one volatile ingredient or volatile preparation as well as stable formulations comprising said microcapsules and application methods using said stable formulations.

Many highly volatile compounds are often present in the formulated products, especially perfuming compounds, which give the formulated products interesting olfactory properties.

Because of their volatile nature, these compounds evaporate rapidly from the formulated product that contains them, which limits their interest since the formulated product thus rapidly loses its olfactory properties.

In addition, some of these compounds are fragile and likely to be degraded as a result of interactions with their environment by mechanisms such as hydrolysis, thermal denaturation or oxidation, which also restricts the life of the olfactory properties of the formulated product.

The encapsulation of volatile compounds represents a very interesting way to limit their evaporation and prevent their degradation, thus increasing the lifetime of the olfactory properties of the formulated product.

A large number of capsules have been developed in order to protect and/or isolate active ingredients in the formulated products, and in particular volatile compounds. These capsules result from manufacturing methods such as spray-drying, interfacial polymerization, interfacial precipitation or solvent evaporation among many others.

However, the diffusion time of volatile compounds through the capsule-forming materials made by most of these methods remains very short, resulting in very rapid leakage of the capsules. Thus, the life of the olfactory properties of the formulated product that contains them is not significantly longer.

The difficulty of providing a truly effective barrier to the diffusion of volatile compounds means that, to date, there is no capsule with satisfactory protection and retention of the properties of these volatile compounds. In other words, the development of capsules with improved protection and retention properties of the volatile compounds remains a constant objective.

Moreover, some of these microcapsules have a shell formed of a non-crosslinked material such as a hydrogel or thermoplastic polymer. If this hydrogel or this thermoplastic polymer is formed of materials known to be biodegradable, then the shell of microcapsules formed of this material will be deemed to be biodegradable. However, diffusion across the shell of this type of capsule is relatively rapid, thereby limiting the performance thereof. An encapsulated compound may quickly escape outside the capsule or, conversely, chemical species degrading the encapsulated compound may quickly enter the capsule.

Other microcapsules have a shell resulting from the reaction of monomers which interact chemically with each other and form a crosslinked material through which diffusion is much slower, thereby improving the performance of the capsules. In this category, mention can be made of capsules formed of urea and formaldehyde which are widely used but unfortunately are not biodegradable.

There is therefore a need for a technique to form capsules formed of a crosslinked shell, have very good retaining and protecting properties for the active ingredients contained therein. There is a further need in some sectors for a technique to form capsules having the aforementioned properties, which are furthermore biodegradable.

The aim of the present invention is thus to provide microcapsules encapsulating at least one volatile ingredient or volatile preparation that reduce the volatility of the volatile ingredient or preparation.

Another aim of the present invention is to provide microcapsules encapsulating at least one volatile ingredient or volatile preparation, with improved biodegradability properties, allowing to reduce their environmental impact and ensure compliance with bans on the intentional use of microplastics.

Therefore, the present invention relates to a solid cross-linked microcapsule encapsulating at least one volatile ingredient or volatile preparation, said microcapsule having a mean diameter from 0.5 $\mu$m to 100 $\mu$m.

The present invention improves the existing microencapsulated formulations of volatile ingredients by providing a formulation that simultaneously reduces the volatility of the ingredient or preparation, allows a good protection and stability of ingredient or preparation in the formulation, controls its release rate over an extended time period after use and is furthermore encapsulated in a biodegradable shell and, if necessary, a biodegradable core additive. When applicable, the microcapsules of the invention have additionally proven equivalent or greater efficacy than their commercial references.

In the present application, the terms « microcapsules » and « capsules » are used indifferently.

The present invention relates to a biodegradable solid cross-linked microcapsule encapsulating at least one volatile ingredient or a preparation comprising a volatile ingredient, said microcapsule having a mean diameter from 0.5 $\mu$m to 100 $\mu$m, said microcapsule comprising:

- a core consisting of a composition C1 comprising at least one volatile ingredient or a preparation comprising a volatile ingredient, and from 1% to 20% by weight of a viscosifying agent relative to the total weight of said composition, and
- a solid cross-linked polymer envelope at least partially encapsulating the core at its periphery,

said solid cross-linked polymer envelope being made of a cross-linked polymer, said polymer being a polymer obtained by polymerizing one or several oligomer(s) or monomer(s),
and wherein the average thickness of said solid cross-linked polymer envelope is from 5% to 30% of the average diameter of said solid cross-linked microcapsule.

The solid microcapsules have a core and a solid envelope (or shell) at least partially encapsulating the core on the periphery thereof, wherein the core consists of a composition C1 comprising at least one volatile ingredient or preparation.

According to the present invention, the core is encapsulated by the shell, said shell forming a surface at the periphery of the core. The core of the microcapsules is totally encapsulated or partially surrounded by the shell.

"At least partially" means that at least 60% of the area of the core surface of the microcapsule is coated (or covered or surrounded) by the solid shell (or shell), preferably 70% of the core surface, and particularly at least 80% of the core surface and even more preferably at least 90% of the core surface.

According to an embodiment, the solid microcapsules have a core and a solid envelope (or shell) totally encapsulating the core on the periphery thereof, wherein the core consists of a composition C1 comprising at least one volatile ingredient or preparation. Preferably, these solid microcapsules are formed of a core containing volatile ingredient(s) or preparation (composition C1) and a solid envelope (or shell) (obtained from a specific polymer) fully encapsulating said core on the periphery thereof.

As mentioned above, the microcapsules are solid cross-linked microcapsules.

The term "shell" refers to a solid cross-linked polymer envelope that has a roughly spherical shape. The function of a shell (or envelope), as used in a microcapsule, is to keep the encapsulated material found within the shell generally separate from the material outside of the microcapsule. The shell (or envelope) may be diffusible so that under appropriate conditions it may allow diffusion into or out of the microcapsule to occur. Another mechanism by which the active may be released is the mechanical rupture of the capsule shell, resulting from the mechanical properties of the shell such as brittleness.

The term "core" of a microcapsule refers to the encapsulated composition located within the shell. The core of the microcapsules according to the invention is made of a composition C1 comprising at least one volatile ingredient or preparation as active agent.

According to the present invention, the term "solid" refers to the non-liquid, cross-linked polymer.

According to the present invention, the term "cross-linked" refers to a polymer bond being formed between two or more oligomers or monomers.

The microcapsules according to the invention have a mean diameter as defined above, said diameter being measured by methods well known to the skilled person in the art, e.g. by a light scattering technique (for example using a Mastersizer 3000 equipped with a hydro SV measuring cell), or by image analysis of optical microscopy pictures, or by image analysis of electronic microscopy pictures.

*Composition C1*

Composition C1 comprises at least one volatile ingredient or preparation as explained above. This composition C1 acts as carrier for the volatile ingredient(s), within the droplets formed during the method of the invention and in the solid capsules obtained.

According to the invention, the term "volatile ingredient" may also be named "active volatile ingredient" or "volatile compound".

According to the invention, the term "volatile preparation" may also be named "volatile composition". This term may also refer to a composition comprising at least one volatile ingredient as defined above.

Preferably, the "active volatile ingredient" is a volatile lipophilic compound.

**Volatile lipophilic compound**

The composition C1 according to the invention comprises at least one volatile lipophilic compound. It may also include a mixture of several volatile compounds.

By "volatile compound" is meant a compound of which a certain volume is capable of rapidly evaporating in less than one hour, at ambient temperature (25°C) and atmospheric pressure (760 mmHg). A volatile compound according to the invention is therefore liquid at ambient temperature, in particular having a non-zero vapor pressure, at ambient temperature and atmospheric pressure, in particular having a vapor pressure ranging from 0.013 Pa to 40 000 Pa ($10^{-4}$ to 300 mm Hg), and preferably from 1.3 Pa at 13 000 Pa (0.01 to 300 mm Hg).

The standard method for determination of vapor pressure for volatiles is defined in the OECD Guideline 104.

Typically, the vapor pressure for the volatile compound, such as volatile compounds in the consumer care industry, is from 0.01 mmHg to 3 mmHg, and preferably from 0.01 mmHg to 2 mmHg.

The active volatile ingredient is preferably selected from the group consisting of: a fragrance (or perfuming agent), a fragrance ingredient, an essential oil, flavonoids, (poly)unsaturated fatty acids, a malodor counteractant, and mixtures thereof.

Typically, the vapor pressure for the volatile compound, such as volatiles of interest in the advanced materials industry, including applications such as flame retardants, catalysts used to polymerize polymer, elastomer formulations, rubber, paint, adhesive, sealant, mortar, varnish or coating, lubricants, accelerators such as crosslinking agents, hardeners, is from 0.01 mmHg to 30 mmHg.

As a flame retardant, one may cite for example perfluoro-2-methyl-3-pentanone. As a catalyst, one may cite tetra-n-butyl-orthotitanate or diisobutoxybisethylacetoacetatotitanate.

According to another embodiment, the volatile lipophilic compounds are chosen from organic solvents such as saturated and unsaturated, halogenated and non-halogenated, linear, branched and cyclic aliphatic hydrocarbons; halogenated and non-halogenated aromatic hydrocarbons; alcohols; glycols such as ethylene glycol, propylene glycol and their derivatives; ketones such as acetone, butanone or methyl isobutyl ketone; esters; linear and cyclic, aliphatic and aromatic ethers, such as methyl t-butyl ether or tetrahydrofuran; glycol ethers such as ethylene glycol monomethyl ether, ethylene glycol monoethyl ether, ethylene glycol monoethyl ether acetate, ethylene glycol monobutyl ether, diethylene glycol monomethyl ether, diethylene glycol monobutyl ether, diethylene glycol monobutyl ether acetate, diethylene glycol monoethyl ether, diethylene glycol monophenyl ether, diethylene glycol dimethyl ether, propylene glycol monomethyl ether, dipropylene glycol monomethyl ether, tripropylene glycol monomethyl ether, propylene glycol monomethyl ether acetate, propylene glycol monoethyl ether acetate, and propylene glycol monobutyl ether.

According to another embodiment, the volatile lipophilic compounds are chosen from flame retardants such as brominated compounds, for example decabromodiphenyl ethers, hexabromocyclododecanes, brominated epoxide oligomers; phosphorus compounds, for example alkyl phosphates, aryl phosphates, bisaryl phosphates; short-chain and medium-chain chloroparaffins (containing up to about 25 carbon atoms).

By "active volatile ingredient", it is also meant here an individual ingredient, or a mixture of ingredients capable of imparting perceptible and desirable benefits to the quality of the air into which they are diffused.

Preferably, the active volatile ingredient is a fragrance, essential oil or a malodor counteractant. Even more preferably, the active volatile ingredient is a fragrance.

According to one embodiment, the volatile lipophilic compounds are chosen from fragrances, flavonoids, (poly)unsaturated fatty acids, and mixtures thereof.

According to the invention, the volatile lipophilic compound may be in the form of a mixture. Thus, the volatile lipophilic compound according to the invention may comprise a single fragrance or a mixture several fragrances.

Among the fragrances, mention may be made of any type of fragrance, these terms being used here indifferently. These fragrances are well known to those skilled in the art and include, in particular, those mentioned, for example, in S. Arctander, Perfume and Flavor Chemicals (Montclair, NJ, 1969), S. Arctander, Perfume and Flavor Materials of Natural Origin (Elizabeth, NJ, 1960), in the International Fragrance Association's list (IFRA http://www.ifraorg.org/en/ingredients) and in "Flavor and Fragrance Materials," 1991 (Allured Publishing Co. Wheaton, III, USA).

The fragrances used in the context of the present invention may include natural products such as extracts, essential oils, absolutes, resinoids, resins, concretes, etc., as well as basic synthetic substances such as hydrocarbons, alcohols, aldehydes, ketones, ethers, acids, esters, acetals, ketals, nitriles, etc., including saturated and unsaturated compounds, aliphatic, alicyclic and heterocyclic compounds.

According to one embodiment, the volatile ingredient comprises less than 10% or even less than 7.5%, by weight of compound(s) with a ClogP less than 3, relative to the total weight of said active. According to one embodiment, the volatile ingredient does not comprise a compound with a ClogP less than 2.1.

According to a preferred embodiment, the volatile ingredient does not comprise a compound with a ClogP less than 1.96.

ClogP refers to the octanol/water partitioning coefficient (P) of active ingredients. The octanol/water partitioning coefficient of an active ingredient is the ratio between its equilibrium concentrations in octanol and in water. The ClogP values reported herein are most conveniently calculated by the "CLOGP" program available within the Chemoffice Ultra Software version 9 available from CambridgeSoft Corporation, 100 CambridgePark Drive, Cambridge, MA 02140 USA or CambridgeSoft Corporation, 8 Signet Court, Swanns Road, Cambridge CB5 8LA UK. The ClogP values are preferably used instead of the experimental logP values in the selection of fragrance ingredients which are useful in the present invention. For natural oils or extracts the composition of such oils can be determined by analysis or using the compositions published in the ESO 2000 database published by BACIS (Boelens Aroma Chemical Information Service, Groen van Prinsterlaan 21, 1272 GB Huizen, The Netherlands).

According to one embodiment, the content by weight of volatile compound(s) (or volatile ingredient(s)) is between 50% and 99%, preferably between 70% and 98%, relative to the weight of the composition C1.

According to an embodiment, composition C1 is monophasic i.e., it is the volatile ingredient(s) alone or it is a solution comprising the volatile ingredient(s) in solubilized form.

In one embodiment, the volatile ingredient(s) is solubilized in composition C1.

In this variant, composition C1 is typically composed of a solution of the volatile ingredient(s) in an aqueous solution or organic solvent, or a mixture of organic solvents, the volatile ingredient(s) being contained in a weight content of between 1% to 99% relative to the total weight of composition C1. The volatile ingredient(s) may be contained in a weight content of between 5% to 95%, 10% to 90%, 20% to 80%, 30% to 70% or 40% to 60% relative to the total weight of composition C1.

In one embodiment, composition C1 consists of the volatile ingredient(s) or preparation.

A "perfuming ingredient" is meant here as a compound which is of current use in the perfumery industry, i.e. a compound which is used as active ingredient in perfuming compositions or in perfumed products in order to impart a hedonic effect into its surroundings. In other words, such an ingredient or mixture, to be considered as being a perfuming one, must be recognized by a person skilled in the art of perfumery as being able to impart or modify, preferably in a positive or pleasant way, the odor of a composition or product, and not just as having an odor. Moreover, this definition is also meant to include compounds that do not necessarily have an odor but are capable of modulating the odor of a perfuming composition or of a perfumed product and, as a result, of modifying the perception by a user of the odor of such a composition or product.

By the term "malodor counteractant" or "malodor counteracting ingredient" is meant compounds which are capable of reducing the perception of malodor, i.e. of an odor that is unpleasant or offensive to the human nose, by counteracting and/or masking malodors. In particular embodiments, these compounds have the ability to react with key compounds which are known or suspected to be the cause of the malodor. The reactions result in reduction of the malodor materials' airborne levels and consequent reduction in the perception of the malodor.

Malodor counteractants can further be defined, based on CA2774661 C, as a freshening composition designed to deliver genuine malodor reduction and not function merely by using perfume to cover up or mask odors. The freshening composition reduces malodor in the air or on inanimate surfaces, for example, fabrics that are contaminated with environmental odors such as food and tobacco odors, or wetted with perspiration. The freshening composition may also reduce microbes on inanimate surfaces or in air. The freshening composition may also act as a barrier to prevent malodors from adhering to or penetrating an inanimate surface.

A genuine malodor reduction provides a sensory and analytically measurable (e.g. gas chromatograph) malodor reduction. Thus, if the freshening composition delivers a genuine malodor reduction, the freshening composition will neutralize malodors in the air and/or on fabrics. "Neutralize" or "neutralization" as used herein means chemically reacting with malodor components (e.g. the reaction of primary amines with aldehydes to form imines, reductive alkylation of amines, protonation and deprotonation of amines, polymerization or de-polymerization); or suppressing the volatility of malodorous components such that other parts of the composition may react (e.g. acid - base neutralization); or physically entrapping odorous compositions.

By way of example, volatile aldehydes such as 2-ethoxy benzylaldehyde, 2-isopropyl-5-methyl-2-hexenal, 5-methyl furfural, 5-methyl-thiophene-carboxaldehyde, adoxal, p-anisaldehyde, benzylaldehyde, bourgenal, cinnamic aldehyde, cymal, decyl aldehyde, floral super, florhydral, helional, lauric aldehyde, ligustral, lyral, melonal, o-anisaldehyde, pino acetaldehyde, P.T. bucinal, thiophene carboxaldehyde, trans-4-decenal, trans trans 2,4-nonadienal, undecyl aldehyde have been cited in the prior art as volatile malodour counteractants.

Volatile ingredients also include flame retardants, such as water.

In another embodiment of the invention, composition C1 is a biphasic composition, which means that the volatile ingredient(s) or volatile preparation is dispersed either in liquid form or in solid form in composition C1 and is not fully solubilized in said composition C1.

As mentioned above, the composition C1 according to the invention comprises a viscosifying agent.

According to an embodiment, the composition C1 further comprises a viscosifying agent as well as a biodegradable carrier oil.

This embodiment reduces the volatility of the volatile ingredient(s) or preparation by encapsulating it in a polymer shell with a high cross-link density and may further include the formulation of a core capsule comprising a biodegradable oil and a viscosifying agent that limits the diffusion of the volatile ingredient(s) or preparation into the shell polymer.

As mentioned above, the composition C1 comprises from 1% to 20%, preferably from 1 % to 15%, and more preferably from 1% to 10%, by weight of a viscosifying agent relative to the total weight of said composition C1.

According to the invention, the term "viscosifying agent" refers to a compound that increases the viscosity of the medium to which it is added, preferably obtaining a viscosity of at least 1,000 mPa.s, more preferably at least 2,000 mPa.s and most preferably above 10,000 mPa.s.

Such agent is thus able to improve the viscosity of the composition C1 containing the volatile ingredient(s) or preparation. A higher viscosity reduces the volatility of the volatile ingredient(s) or preparation from the capsule by limiting the diffusion of the volatile ingredient(s) or preparation into the polymer envelope as defined above.

Preferably, the viscosifying agent is selected from the group consisting of: fumed silica, clay, organic polymers such as hydrogenated vegetable oils, polyesters, such as polyester block co-polymers, hydrogenated styrene/isoprene copolymer, polyamides, polyurethanes, oil-gelling polymers, hydrogels, such as polysaccharides or hydrophilic silicates, and mixtures thereof.

According to a preferred embodiment, the viscosifying agent is fumed silica.

According to an embodiment, the composition C1 further comprises a carrier oil, said carrier oil being preferably biodegradable.

According to the present invention, the term "oil" refers to a fatty compound or substance which is in the form of a liquid or paste (not solid) at room temperature (25°C) under atmospheric pressure (760 mmHg). These oils may be volatile or non-volatile.

Among the oils usable in the present invention, we may cite: volatile or non-volatile oils; these oils may be hydrocarbon oils, particularly of animal or vegetable origin, synthetic oils, silicone oils, fluorinated oils or mixtures thereof.

By "hydrocarbon oil" or "hydrocarbon-based oil" it is meant an oil containing mainly hydrogen and carbon atoms and optionally oxygen, nitrogen, sulfur and/or phosphorus atoms. Hydrocarbon oil does not include silicon atoms.

By "silicone oil" it is meant an oil comprising at least one silicon atom, and in particular at least one Si-O group.

By "fluorinated oil" it is meant an oil comprising at least one fluorine atom.

Preferably, hydrocarbon oils can be selected from:

- linear or branched, optionally cyclic, C6-C16 lower alkanes. Examples include hexane, undecane, dodecane, tridecane and isoparaffins such as isohexadecane, isododecane and isodecane;
- linear or branched hydrocarbons containing more than 16 carbon atoms, such as vegetable oils, including but not limited to triglyercides and functionalized triglycerides; kerosene oils, liquid petroleum jelly, polydecenes and hydrogenated polyisobutenes such as Parleam and squalane; or
- alkane mixtures, e.g. C9-12 Alkane, C10-13 Alkane, C13-14 Alkane, C13-15 Alkane, C14-17 Alkane, C14-19 Alkane, C15-19 Alkane, C15-23 Alkane, C18- 21 Alkane, C8-9 Alkane/Cycloalkane, C9-10 Alkane/Cycloalkane, C9-11 Alkane/Cycloalkane, C9-16 Alkane/Cycloalkane, C10-12 Alkane/Cycloalkane, C11-14 Alkane/Cycloalkane, C11-15 Alkane/Cycloalkane, C12-13 Alkane/Cycloalkane.

Examples of synthetic oils include alkane oils such as isododecane and isohexadecane, ester oils, ether oils and artificial triglycerides.

According to a preferred embodiment, the composition C1 further comprises a biodegradable carrier oil.

According to an embodiment, the composition C1 further comprises from 5% to 80%, preferably from 5% to 40% by weight of a carrier oil, preferably a biodegradable oil, relative to the total weight of said composition C1.

More preferably, the composition C1 comprises from 5% to 30% by weight of a carrier oil, preferably a biodegradable oil, relative to the total weight of said composition C1.

According to the invention, the term "biodegradable carrier oil" refers to an oil in which the volatile ingredient(s) or preparation is miscible, which is furthermore biodegradable as defined by OECD Test 301.

Preferably, the biodegradable carrier oil is selected from the triglyceride oils and more preferably from the polyester oils.

As preferred polyester oils, one may cite those that are liquid at ambient temperature (from 20°C-50°C), and that have a boiling point above 60°C.

The biodegradable carrier oil according to the present invention has preferably a viscosity at room temperature comprised from 20 mPa.s to 5,000 mPa.s, more preferably from 200 mPa.s to 5,000 mPa.s.

As preferred carrier oils, one may cite unsaturated polyol esters such as, by way of example, DEHYLUB® 4038 from EmeryOleo.

Alternatively, natural oils and natural lipid derivatives can be used as the biodegradable carrier oil, including but not limited to almond oil, castor oil, hydrogenated castor oil, fish oil, linseed oil, palm oil, hydrogenated palm oil, wheat germ oil, corn oil, cottonseed oil, hydrogenated cottonseed oil, sesame oil, soybean oil, hydrogenated soybean oil, butyl stearate, glyceryl monooleate, isopropyl myristate, oleic acid, dimer dilinoleyl dimer dilinoleate, cetearyl isononanoate, coco-caprylate/caprate, decyl oleate octyldodecyl myristate, ethylhexyl stearate, pentaerythrityl tetraisostearate, polymer of 12-hydroxystearic acid and 2-ethyl hexyl alcohol (ethylhexyl polyhydroxystearate), and/or combinations thereof.

According to a preferred embodiment, the composition C1 comprises from 20% to 100%, such as from 20% to 80%, in particular from 30% to 80%, preferably from 40% to 80%, more preferably from 50% to 80%, and even more preferably from 60% to 80%, by weight of volatile ingredient(s) or preparation product relative to the total weight of said composition.

## Polymer envelope

In one embodiment, the above-mentioned solid microcapsules comprise a solid shell that is entirely composed of cross-linked polymer. This polymer may also be named copolymer or be a (co)polymer network.

The microcapsules according to the invention comprise a shell or envelope as defined above that is made of a cross-linked polymer, said polymer being a polymer, preferably an aliphatic polymer, obtained by polymerizing one or several oligomer(s) or monomer(s).

According to another embodiment, the cross-linked polymer is an aliphatic polymer.

According to one embodiment, said polymer is a biodegradable cross-linked polymer.

In the invention, the term « monomer », « oligomer » or « polymer » designates any base unit adapted for the formation of a solid material via polymerization, either alone or in combination with other monomers or polymers. The term « polymer » also encompasses oligomers.

The average thickness of the polymer envelope as defined above is measured by optical microscopy, wherein a collection of capsules is observed through an optical microscope and the image is recorded digitally. Standard digital tools available to a person skilled in the art of microscopy such as imaged can be utilized to determine the thickness of the polymer envelope based on the contrast between the envelope and core. A minimum of 10 and preferably a minimum of 20 microcapsules can be analyzed in this manner to obtain an average value.

According to an embodiment, the microcapsules according to the invention comprise a shell or envelope as defined above that is made of a biodegradable cross-linked polymer, said polymer being an aliphatic polymer, obtained by polymerizing one or several oligomer(s) or monomer(s).

According to the invention, the terms "aliphatic" and "nonaromatic" are used indifferently.

Preferably, the microcapsules according to the invention comprise a shell or envelope as defined above that is made of a biodegradable cross-linked polymer, said polymer being a polymer obtained by polymerizing one or several oligomer(s) or monomer(s), said polymer comprising at least 20%, in particular at least 25%, and more preferably at least 30%, of ester groups by weight in comparison with the total weight of said polymer

Preferably, the microcapsules according to the invention comprise a shell or envelope as defined above that is made of a biodegradable cross-linked polymer, said polymer being an aliphatic polymer obtained by polymerizing one or several oligomer(s) or monomer(s), said polymer comprising at least 20%, in particular at least 25%, and more preferably at least 30%, of ester groups by weight in comparison with the total weight of said polymer

According to a another embodiment, the microcapsules according to the invention comprise a shell or envelope as defined above that is made of a biodegradable cross-linked polymer, said polymer is a polymer obtained by polymerizing one or several oligomer(s) or monomer(s), said polymer comprising at least 20%, in particular at least 25%, and more preferably at least 30%, of ester groups by weight in comparison with the total weight of said polymer.

Throughout the present application, biodegradability is defined herein as the ability to degrade in a natural medium such as defined in OECD standards: OECD 301 (Ready biodegradability), namely OECD 301 A (Dissolved Organic Carbon (DOC) Die-Away), OECD 301 B ($CO_2$ Evolution), OECD 301 C (Modified MITI (I) test), OECD 301 D (Closed Bottle test), OECD 301 E (Modified OECD Screening), OECD 301 F (Manometric Respirometry test), or further in OECD 304A (Inherent Biodegradability in Soil), OECD 306 (Biodegradability in Seawater) and OECD 310 (Ready Biodegradability - $CO_2$ in Sealed Vessels), and OECD NF T 51800 (Compostable products definition).

The suitability of the microcapsule for encapsulation of the volatile ingredient(s) or preparation is connected to certain attributes of the capsule, such as its core composition and the chemistry of the shell. In particular, controlling the core viscosity by controlling the composition of the core enables an improved retention of the volatile ingredient(s) or preparation. Furthermore, the chemistry of the capsule shell also has an impact on the retention of the volatile ingredient(s) or preparation. In particular, the percentage of ester groups in the shell and the molecular weight of the spacers are parameters that may have an impact on the retention of the volatile ingredient(s) or preparation. The spacer is defined as the distance between the ester groups in the final polymer shell (or envelope).

According to an embodiment, the polymer envelope comprises an aliphatic (co)polymer network of one or more oligomer(s) or monomer(s) comprising at least 25% weight of ester groups, preferably at least 30% weight, in relation to the total weight of said polymer. The primary component of the network can be referred to as M1 (or oligomer M1) and the secondary component can be referred to as M2 (monomer or oligomer M2). The distinction between M1 and M2 is for example the molecular weight. Preferably, the molecular weight of M1 is from 1,500 to 10,000 g/mol and the molecular weight of M2 is from 50 to 1,500 g/mol.

According to a preferred embodiment, the aliphatic polymer of the solid cross-linked polymer envelope is obtained by polymerizing one or several oligomer(s) or monomer(s) M1 and M2, the molecular weight of M1 being from 1,500 to 10,000 g/mol and the molecular weight of M2 being from 50 to 1,500 g/mol, said oligomers M1 and M2 comprising preferably at least one reactive function selected from the group formed by acrylate, methacrylate, vinyl ether, N-vinyl ether, epoxy, siloxane, amine, lactone, phosphate and carboxylate functions.

According to a more preferred embodiment, the aliphatic polymer of the solid cross-linked polymer envelope is obtained by polymerizing one or several oligomer(s) or monomer(s) M1 and M2, the molecular weight of M1 being from 1,500 to 10,000 g/mol and the molecular weight of M2 being from 50 to 1,500 g/mol, said oligomers M1 and M2 comprising preferably at least one reactive function selected from the group formed by acrylate, methacrylate, lactone and carboxylate functions.

Preferably, the shell (or envelope) chemistry is defined through the minimal percentage of ester groups in the shell, which has a high influence on the biodegradability and on the cross-linking density of said shell. It will be clear to a person skilled in the art that the selection of the oligomers and monomers that are contained in the envelope determines the value of the average molecular weight of the spacer between the cross-links (or ester groups). By way of example, the spacer between the cross-links can be defined as a chain between two ester groups. To produce capsules comprising said polymer network in the shell, the raw materials can be selected using the fact that this value can be calculated by determining the average of the ratio of the molecular weight of the oligomer/(number of reactive function(s)-1)*% in the pre-polymerized oligomer(s) M1 and M2. The value may also be determined experimentally from the final polymerized solid envelope in the following manner: acid hydrolysis can be used to break down the ester bonds after which a chemical analysis of the molecular weight of the constituents between each ester bond can be performed using methods known to a person skilled in the art such as size exclusion chromatography (SEC) or TOF-SIMS. SEC may be carried out with a Waters 510 HPLC pump equipped with three columns from Polymer Labs, Inc., having 5 $\mu$m bead size (two with MIXED-D and one 50 Å pore sizes). THF can be used as the eluent. Such a measurement system is typically equipped with a Waters R401 differential refractometer as the detector. Typically the weight fraction distribution as a function of molecular weight can be determined, which provides a value for the distance between the ester bonds in the polymer envelope.

The reactive functions as mentioned above in M1 and/or M2 are preferably selected from the group formed by acrylate, methacrylate, vinyl ether, N-vinyl ether, epoxy, siloxane, amine, lactone, phosphate and carboxylate functions.

The influence of selection of pre-polymerized oligomers on the percentage of ester groups in the final solid polymerized shell also plays a role in the properties of the capsule and its performance. The selection of oligomers M1 and M2 can be optimized by tailoring the selection thereof to achieve a minimum of 25% weight of ester groups.

The percentage of ester groups in a polymerized shell is the ratio between the weight of ester groups present in the shell and the weight of total polymer shell material. One can determine this value from the pre-polymerized oligomers M1 and M2 with the following formula:

$$\sum_{oligomers\ i} f_i \frac{n_{ester\ i}}{M_{wi}} M_{Wester}$$

where:

-  the sum considers all oligomers irrespective of their type M1 or M2,
-  $f_i$ is the number percentage of oligomer i in the shell material,
-  $n_{ester\ i}$ is the sum of the number of ester groups and reactive functions that subsequently become an ester group after cross-linking within oligomer $I$,
-  $M_{wi}$ is the molecular weight of oligomer $I$, and
-  $M_{w\ ester}$ is the molecular weight of an ester group.

Furthermore, the % ester groups can also be determined experimentally from the final polymerized solid envelope in the following manner: using solid nuclear magnetic resonance methods, by which the ester atoms have a specific resonant frequency, which can be compared to the resonant frequency of the atoms in a carbon-carbon bond through a ratio between the two, the ratio thereof thereby providing a percentage of the ester groups in the total molecular structure of the shell. In particular the peak observed using $^{13}$C MAS NMR, at 180 ppm is characteristic of an ester bond, whereas a C-H bond is characterized by a peak at 30 ppm. Solid state $^{13}$C MAS-NMR proton decoupling single-pulse spectra of polymers can be obtained with a Bruker Avance DRX- 400 spectrometer using a magnetic field of 9.36 T and equipped with a multinuclear probe. The measurement conditions are preferably as follows: Minced samples are packed in 4 mm Ø zirconia rotors and spun at 10 KHz. The spectra are acquired at a frequency of 100.61 MHz, using a $\pi$/6 pulse width of 2.5 $\mu$s and a pulse space of 10 s to ensure full relaxation and to allow quantitative analysis from peak areas. Such methods are detailed in for instance Benítez, Jose & Heredia-Guerrero, José & Guzman-Puyol, Susana & Barthel, Markus & Dominguez, Eva & Heredia, Antonio. (2015). "Polyhydroxyester Films Obtained by Non-Catalyzed Melt-Polycondensation of Natural Occurring Fatty Polyhydroxyacids." Frontiers in Materials. 2. 10.3389/fmats.2015.00059.

Among the examples of such oligomers for M1, the following compounds and mixtures thereof may be mentioned: the family of aliphatic esters and polyesters particularly comprising polyglycolides (PGAs), polylactides (PLAs), poly(lactide-co-glycolide) (PLGAs), poly(ortho esters) e.g. polycaprolactone (PCL), polydioxanone, polyethylene succinate), poly(butylene succinate) (PBS), polyethylene adipate), poly(butylene adipate), poly(ethylene sebacate), poly(valerolactone) (PVL), poly(decalactone), polyhydroxyvalerate, poly(beta-malic acid), poly-3-hydroxybutyrate (PHB), poly-3-hydroxybutyrate-co-3-hydroxyvaldrate (P-3H B-3HV), poly-3-hydroxybutyrate-co-4-hydroxybutyrate (P-3H B-4H B), poly-3-hydroxybutyrate-co-3-hydroxyvalerate-co-4-hydroxybutyrate (P-3HB-3HV-4HB), poly (3-hydroxyvalerate), poly(3-hydroxypropionate), poly (3-hydroxycaproate), poly (3-hydroxyoctanoate), poly(3-hydroxydecanoate), poly(3-hydroxyundecanoate), poly(3-hydroxydodecanoate), poly(3-hydroxybutyrate-co-3-hydroxyvalerate), poly(3-hydroxybutyrate-co-3-hydroxydecanoate), poly(3-hydroxybutyrate-co-3-hydroxypropionate), poly(3-hydroxybutyrate-co-3-hydroxyoctanoate), poly(3-hydroxyheptanoate), poly(3-hydroxyhexanoate), poly(2-hydroxybutyrate), poly(3-hydroxybutyrate-co-4-hydroxy-butyrate), poly(3-hydroxybutyrate-co-3-hydroxyhexanoate), poly(3-hydroxybutyrate-co-3-hydroxyhexanoate), poly(4-hydroxybutyrate), poly(4-hydroxybutyrate-co-2-hydroxybutyrate), poly(4-hydroxypropionate), poly(4-hydroxyvalerate), poly(5-hydroxybutyrate), poly(5-hydroxyvalerate), poly(6-hydroxyhexanoate), poly(alkylene alkanoate), poly(alkylene dicarboxylate), poly(butylene adipate, poly(butylene carbonate), poly(butylene pimelate), poly(butylene succinate), poly(butylene succinate-co-adipate), poly(butylene succinate-co-carbonate), poly(butylene sebacate), poly(butylene succinate-co-lactate), polydiaxanone, polyethylene azelate), polyethylene carbonate), polyethylene decamethylate), polyethylene furanoate), polyethylene oxalate), polyethylene succinate), polyethylene succinate-co-adipate), polyethylene sebacate), polyethylene suberate), poly(hexamethylene sebacate), poly(glycolide-co-caprolactone), poly(lactide-co-epsilon-caprolactone), polymandelide, poly (B-malic acid), poly(propylene succinate), poly(tetramethylene carbonate), poly(trimethylene carbonate), poly(tetramethylene succinate)-co-(tetramethylene carbonate), poly(trimethylene adipate), poly(tetramethylene adipate), poly(tetramethyl glycolide), aliphatic poly(urethane) such as polycaprolactone, polycarbonate urethanes, aliphatic urethane diacrylate, aliphatic urethane methacrylate, aliphatic urethane triacrylate, non-isocyanate polyurethanes, poly(valerolactone), additionally carrying at least one reactive function selected from the group formed by acrylate, methacrylate, vinyl ether, N-vinyl ether, epoxy, siloxane, amine, lactone, phosphate and carboxylate functions.

Among the examples of oligomers for M2, the following compounds and mixtures thereof may be mentioned: diacrylates e.g. 1,6-hexanediol diacrylate, 1,6-hexanediol dimethacrylate, polyethylene glycol diacrylate, polyethylene glycol dimethacrylate, 1,9-nonanediol dimethacrylate, 1,4-butanediol dimethacrylate, 1,3-butanediol dimethacrylate, 1,10-decanediol dimethacrylate, bis(2-methacryloxyethyl) N,N'-1 ,9-nonylene biscarbamate, 1,4-butanediol diacrylate, 1,5-pentanediol dimethacrylate, allyl methacrylate, N,N'-methylenebisacrylamide, 2,2-bis[4-(2-hydroxy-3-methacryloxypropoxy)phenyl]propane, tetraethylene glycol diacrylate, diethylene glycol diacrylate, triethylene glycol diacrylate, triethylene glycol dimethacrylate, polyethylene glycol diglycidyl ether, N,N-diallylacrylamide or glycidyl methacrylate; multifunctional acrylates e.g. dipentaerythritol pentaacrylate, 1,1,1-trimethylolpropane triacrylate, 1,1,1-trimethylolpropane trimethacrylate, ethylenediamine tetramethacrylate, pentaerythritol triacrylate or pentaerythritol tetraacrylate; and acrylates also having another reactive function e.g. propargyl methacrylate, N-acryloxysuccinimide, N-(2-Hydroxypropyl) methacrylamide, N-(t-BOC-aminopropyl) methacrylamide, monoacryloxyethyl phosphate, acrylic anhydride, 2-(tert-butylamino) ethyl methacrylate, N,N-diallylacrylamide , N-ethoxy ethyl acrylates, propoxylated N- glyceryl triacrylate or glycidyl methacrylate or ethers and polyethers particularly comprising polyethylene glycols, additionally carrying at least one reactive function selected from the group formed by acrylate, methacrylate, vinyl ether, N-vinyl ether, epoxy, and amine functions and mixtures thereof.

According to a preferred embodiment, the solid cross-linked microcapsule according to the invention is devoid of surfactant. Preferably, neither the core nor the envelope comprises any surfactant.

According to a preferred embodiment, the solid cross-linked microcapsule according to the invention further comprises an anionic polyelectrolyte polymer, preferably a lignosulfonate. Preferably, said anionic polyelectrolyte polymer is in the envelope of the microcapsules. Said preferred embodiment improves the dispersion of microcapsules in a liquid slurry.

According to an embodiment, the present invention relates to a solid cross-linked microcapsule as defined above, wherein the solid cross-linked polymer envelope is formed through polymerization.

Preferably, the solid cross-linked polymer envelope is formed through UV polymerization.

The encapsulation systems in the prior art are based on either interfacial polymerization or coacervation, whereas the present invention preferably uses a double emulsion method that allows better control of capsule geometry, size distribution and enables the use of a greater diversity of shell chemistries. It may furthermore reduce energy consumption and waste produced over the course of capsule production.

Preferably, the solid cross-linked microcapsule is prepared according to the specific process as explained hereafter, such as the one disclosed in WO 2018/0210857.

The process for the preparation of a solid cross-linked microcapsule according to the invention may for example comprise the following steps:

a) under stirring, adding a composition C1 comprising the volatile ingredient(s) or preparation to a polymeric composition C2, compositions C1 and C2 not being miscible with each other, C1 the composition C1 being as defined above,

the viscosity of composition C2 being between 500 mPa.s and 100 000 mPa.s at 25°C, and preferably being higher than the viscosity of composition C1,
the composition C2 comprising:

- one or several oligomer(s) or monomer(s) M1 and M2 as defined above, and
- at least one photoinitiator, crosslinking catalyst,

after which an emulsion (E1) is obtained comprising droplets of the composition C1 dispersed in the composition C2;

b) under stirring, adding the emulsion (E1) to a composition C3, compositions C2 and C3 not being miscible with each other,

the viscosity of composition C3 being between 500 mPa.s and 100 000 mPa.s at 25° C, and preferably being higher than the viscosity of emulsion (E1),
after which a double emulsion (E2) is obtained comprising droplets dispersed in composition C3;

c) applying shear to emulsion (E2),
after which a double emulsion (E3) is obtained comprising droplets of controlled size dispersed in composition C3; and
d) polymerizing composition C2, after which solid cross-linked microcapsules are obtained dispersed in composition C3.

In one embodiment, at step a), the composition C1 is added to a crosslinkable polymeric composition C2, this step being conducted under stirring which means that composition C2 is kept under agitation typically mechanically whilst composition C1 is added, to emulsify the mixture of compositions C1 and C2.

Throughout step a), composition C1 is at a temperature of between 0°C and 100°C, preferably between 10°C and 80°C, and more preferably between 15°C and 60°C. Throughout step a), composition C2 is at a temperature of between 0°C and 100°C, preferably between 10°C and 80°C, and more preferably between 15°C and 60°C.

Under the conditions for addition at step a), compositions C1 and C2 are not miscible with each other, which means that the amount (by weight) of composition C1 capable of being solubilized in C2 is equal to or lower than 7 %, preferably lower than 1 %, and more preferably lower than 0.5 % relative to the total weight of composition C2, and that the amount (by weight) of composition C2 capable of being solubilized in composition C1 is equal to or lower than 10 %, preferably lower than 1 %, and more preferably lower than 0.5 % relative to the total weight of composition C1.

Therefore, when composition C1 comes into contact with C2 under agitation, it is dispersed in the form of droplets called single droplets.

The immiscibility between compositions C1 and C2 also allows prevented migration of the volatile ingredient(s) or preparation of composition C1 towards composition C2.

Composition C2 is stirred to form an emulsion comprising droplets of composition C1 dispersed in composition C2. This emulsion is also called a « single emulsion » or C1-inC2 emulsion.

To carry out step a), it is possible to use any type of mixer usually used to form emulsions e.g. a mechanical blade mixer, static emulsifier, ultrasonic homogenizer, membrane homogenizer, high-pressure homogenizer, colloidal mixer, high-shear disperser or high-speed homogenizer.

In one embodiment, the ratio between the volume of composition C1 and the volume of composition C2 varies between 1:10 and 10:1. Preferably, this ratio is between 1:3 and 5:1, more preferably between 1:3 and 3:1.

Preferably, the viscosity of composition C2 at 25° C is between 1 000 mPa.s and 300 000 mPa.s, more preferably between 25 000 mPa.s and 250 000 mPa.s, for example it is between 50 000 mPa.s and 25 000 mPa.s.

Preferably, the viscosity of composition C2 is higher than the viscosity of composition C1.

Viscosity is measured using a Haake Rheostress™ 600 rheometer equipped with cone of diameter 60 mm having 2-degree angle, and a temperature control cell set at 25° C. The value of viscosity is determined at a shear rate of 10 s$^{-1}$.

In this embodiment, the destabilizing kinetics of the droplets of emulsion (E1) are significantly slow, allowing the shell of the microcapsules to be polymerized at step d) before the emulsion become unstable. Polymerization, once completed, then provides thermodynamic stabilization. Therefore, the relatively high viscosity of composition C2 ensures the stability of emulsion (E1) obtained after step a).

Composition C2 contains at least one monomer or oligomer such as defined below, and at least one photoinitiator or crosslinking catalyst, making the composition crosslinkable.

In one embodiment, composition C2 comprises from 50% to 99% by weight of monomer or polymer such as defined below, or a mixture of monomers and polymers such as defined below, relative to the total weight of composition C2.

In one embodiment, composition C2 comprises from 0.1% to 5% by weight of photoinitiator or a mixture of photoinitiators, relative to the total weight of composition C2.

By « photoinitiator » it is meant a compound capable of fragmenting under the effect of light radiation.

The photoinitiators which can be used in the present invention are known in the art and are described for example in *"Les photoinitiateurs dans la reticulation des revêtements"* (Photoinitiators in the crosslinking of coatings) G. Li Bassi, Double Liaison - Chimie des Peintures, N°361, November 1985, p.34-41 ; *"Applications industrielles de la polymérisation photoinduite"* (Industrial applications of photoinduced polymerization) Henri Strub, L'Actualité Chimique, February 2000, p.5-13; and *"Photopolymères : considérations théoriques et réaction de prise"* (Photopolymers: theoretical considerations and curing reaction), Marc, J.M. Abadie, Double Liaison - Chimie des Peintures, N°435-436, 1992, p.28-34.

At step b) of the method of the invention, a second emulsion (E2) is prepared.

The second emulsion is composed of a dispersion of droplets of the first emulsion in a composition C3 immiscible with C2. Said emulsion E2 may be created through the addition of emulsion (E1) to C3 under stirring. Said addition may be dropwise.

Throughout step b), emulsion (E1) is at a temperature of between 15°C and 60°C. Throughout step b), composition C3 is at a temperature of between 15°C and 60°C.

Under the conditions for addition at step b), compositions C2 and C3 are not miscible with each other, which means that the amount (by weight) of composition C2 capable of being solubilized in composition C3 is equal to or lower than 5%, preferably lower than 1%, and more preferably lower than 0.5% relative to the total weight of composition C3, and that the amount (by weight) of composition C3 capable of being solubilized in composition C2 is equal to or lower than 5%, preferably lower than 1%, and more preferably lower than 0.5% relative to the total weight of composition C2.

Therefore, when emulsion (E1) comes into contact with composition C3 under agitation, it is dispersed in the form of droplets called double droplets, the dispersion of these droplets of emulsion (E1) in the C3 continuous phase being called emulsion (E2).

Typically, a double droplet formed at step b) corresponds to a single droplet of composition C1 such as described above, surrounded by a shell of composition C2 which fully encapsulates said single droplet.

The double droplet formed at step b) may also comprise at least two single droplets of composition C1, said single droplets being surrounded by a shell of composition C2 which fully encapsulates said single droplets.

Therefore, said double droplets comprise a core composed of one or more single droplets of composition C1, and a layer of composition C2 surrounding said core.

The resulting emulsion (E2) is generally a monodisperse double emulsion (C1-in C2-in C3 emulsion, or C1/C2/C3 emulsion), which means that the double droplets do not have a distinct size distribution in emulsion (E2).

The immiscibility between compositions C2 and C3 allows prevented mixing between the layer of composition C2 and composition C3, and thereby ensures the stability of emulsion (E2).

The immiscibility between compositions C2 and C3 also allows prevented migration of the water-soluble substance of C1 from the core of the droplets towards composition C3.

To implement step b), it is possible to use any type of mixer usually used to form emulsions, e.g. a mechanical blade mixer, static emulsifier, ultrasonic homogenizer, membrane homogenizer, high-pressure homogenizer, colloidal mixer, high-shear disperser or high-speed homogenizer.

In one embodiment, the viscosity of composition C3 at 25°C is higher than the viscosity of emulsion (E1) at 25°C.

In the invention, the viscosity of composition C3 at 25°C is between 500 mPa.s and 100 000 mPa.s.

Preferably, the viscosity of composition C3 at 25°C is between 3 000 mPa.s and 100 000 mPa.s, more preferably between 5000 mPa.s and 80 000 mPa.s, e.g. between 7000 mPa.s and 70 000 mPa.s.

In this embodiment, given the very high viscosity of the continuous phase formed by composition C3, the rate of destabilization of the double droplets of emulsion (E2) is significantly slow compared with the duration of the method of the invention, which therefore affords kinetic stabilization of emulsion (E2) and then of (E3) until polymerization of the shell of the capsules is completed. Once polymerized, the capsules are thermodynamically stable.

Therefore, the very high viscosity of composition C3 ensures the stability of emulsion (E2) obtained after step b).

Low surface tension between C3 and the first emulsion as well as high viscosity of the system advantageously allow ensured kinetic stability of the double emulsion (E2), preventing dephasing thereof throughout the production time.

Preferably, the interfacial tension between compositions C2 and C3 is low. This low interfacial tension between compositions C2 and C3 also advantageously allows ensured stability of emulsion (E2) obtained after step b).

The volume fraction of the first emulsion in C3 can be varied between 0.05 and 0.5 % first to improve production yield and secondly to vary the mean diameter of the capsules. On completion of this step, the size distribution of the second emulsion is relatively monodisperse.

In one embodiment, the ratio between the volume of emulsion (E1) and the volume of composition C3 varies from 1:10 to 10:1. Preferably, this ratio is from 1:9 to 3:1, more preferably from 1:9 to 1:1.

In one embodiment, the composition C3 further comprises one or several water-soluble anionic polyelectrolyte polymer(s). The composition C3 may comprise from 0.01 to 15 mass% of said polymer, preferably from 0.05 to 10 mass% of said polymer, more preferably from 0.1 to 10% of said polymer. In one embodiment, said anionic polyelectrolyte polymer is a lignosulfonate.

Preferably, in this embodiment, the second emulsion is added continuously to the mixer, which means that the amount of double emulsion (E2) fed into the mixer is the same as the amount of third emulsion (E3) leaving the mixer.

Since the size of the droplets of emulsion (E3) essentially corresponds to the size of the droplets of the solid microcapsules after polymerization, it is possible to adjust the size of the microcapsules and the thickness of the shell by adjusting the shear rate at step c), with strong correlation between the reduction in size of the droplets and the increase in shear rate. This correlation is also precisely described in the international application WO 2022/117681.

At step d) of the method of the invention, the shell of the solid microcapsules of the invention is cross-linked and hence formed.

This step allows both expected performance levels to be reached for capsule retention and ensured thermodynamic stability thereof, by definitively preventing any destabilization mechanism such as coalescence or maturation.

In one embodiment, when composition C2 comprises a photoinitiator, step d) is a photopolymerization step whereby emulsion (E3) is exposed to a light source able to initiate photopolymerization of composition C2, in particular to a UV light source preferably emitting in the wavelength range of between 100 nm and 400 nm, and in particular for a time of less than 15 minutes.

In this embodiment, at step d) emulsion (E3) is subjected to photopolymerization, which will allow photopolymerization of composition C2. This step will allow the obtaining of microcapsules encapsulating the water-soluble substance such as defined above.

In one embodiment, at step d) emulsion (E3) is exposed to a light source able to initiate photopolymerization of composition C2.

Preferably, the light source is a UV light source.

In one embodiment, the UV light source emits in the wavelength range of between 100 nm and 420 nm.

In one embodiment, emulsion (E3) is exposed to a light source for a time of less than 15 minutes, preferably for 30 seconds to 10 minutes.

At step d), the shell of the above-mentioned double droplets composed of photo-cross-linkable composition C2, is cross-linked and thereby converted to a viscoelastic polymeric shell encapsulating and protecting the water-soluble substance against release thereof in the absence of mechanical triggering.

In another embodiment, when composition C2 does not comprise a photoinitiator, step d) is a polymerization step without exposure to a light source.

The composition obtained after step d), comprising solid microcapsules dispersed in composition C3, is ready for use and can be used without any additional post-treatment step of the capsules being required.

The present invention also relates to formulations comprising at least one solid cross-linked microcapsule as defined above.

These formulations are advantageous in that they are stable.

The present invention also relates to the use of a solid cross-linked microcapsule as defined above, for reducing the volatility of the volatile ingredient(s) or preparation.

The present invention also relates to a method for reducing the volatility of the volatile ingredient(s) or preparation, comprising the encapsulation of the volatile ingredient(s) or preparation into a solid cross-linked microcapsule as defined above.

The present invention also relates to the use of a formulation comprising at least one solid cross-linked microcapsule according to the invention in home care such as laundry care and cleaning products, home care, personal care such as cosmetics, or hair care.

The present invention also relates to the use of a formulation comprising at least one solid cross-linked microcapsule according to the invention in electronics, construction materials, or textile functionalization.

The microcapsules of the invention can be formulated alone or in mixtures comprising further active ingredients in addition to the volatile ingredient(s) as defined above. Alternatively, the formulation of the invention may comprise the volatile ingredient(s) or volatile preparation as only active ingredient and then be mixed with further actives ingredients. Thus, the formulations of the invention may comprise the microcapsules containing the volatile ingredient(s) or preparation as described herein and at least one additional volatile active ingredient or be mixed with said at least one additional volatile active ingredient.

The term "a" or "an" as used herein includes the singular and the plural, unless specifically stated otherwise. Therefore, the terms "a," "an" or "at least one" can be used interchangeably in this application.

For purposes of better understanding the present teachings and in no way limiting the scope of the teachings, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques. In this regard, used of the term "about" herein specifically includes ±5% from the indicated values in the range. In addition, the endpoints of all ranges directed to the same component or property herein are inclusive of the endpoints, are independently combinable, and include all intermediate points and ranges.

The expressions « between ... and ... », « from ... to ... » and « ranging from ... to ... » are to be construed as including the limits unless specified otherwise.

## EXAMPLES

### Example 1 - **Preparation of microcapsules comprising a fragrance**

A mechanical stirrer (Ika Eurostar 20) equipped with an impeller of disperser blade type was used for all mixing steps.

### Step a): Creation of the Core of the Capsules (Dispersion of Particles - Composition C1)

The core of the capsule is obtained by mixing the above components at 50 °C until homogeneous, thereby obtaining C1.

### Step b): Preparation of the shell composition C2

The shell of the capsule is prepared by mixing the below components at RT at a speed of between 30-70 RPM until homogeneous, thereby obtaining C2.

### Step c) Preparation of the first emulsion (E1)

The composition of the core C1 is added at RT to the composition of the shell C2 with a mixing rate of 70 rpm to produce the first emulsion E1. The ratio of the weight of the shell C2 to the weight of the core C1 is 40/60. An emulsion C1-in-C2 is formed.

### Step d) Preparation of the second emulsion (E2)

Composition C3, the continuous phase is produced by mixing of the components of C3 listed below, at 2,000 rpm until complete homogenization. The first emulsion E1 is then added to the composition C3, which is then stirred at 2,000 rpm for 2 minutes at RT to obtain a monodisperse second emulsion E2, at a ratio of E1 :C3 of 10:90.

### Step e) Reticulation of the Capsule Envelope

The second emulsion (E2) obtained in the previous step is irradiated for 2 minutes with the aid of a UV light source (Dymax LightBox ECE 2000) having a maximum light intensity of 1 W/cm2 at a waveform length of 365 nm.
The microcapsules were separated from their continuous phase by centrifugation at 2000 G for over 3 cycles for a total of 20 min.

| | Raw materials | % in composition |
|---|---|---|
| **Composition C1 Liquid Core** | Volatile Active (Fragrance Composition) | 60 |
| | Priolube 1847 | 37 |
| | Fumed silica | 3 |
| **Total** | | 100 |

| | Raw materials | % in composition |
|---|---|---|
| **Composition C1 Viscose Core** | Volatile Active (Fragrance Composition) | 60 |
| | Viscous Carrier oil (Esters - Complex ester, Priolube 1847 ) | 23 |
| | Viscosifying agent (Caprylic/Capric Triglyceride (and) Polyurethane-79) | 17 |
| **Total** | | 100 |

| | Raw materials | % |
|---|---|---|
| **Composition C2** | Oligomer (Aliphatic Urethane Acrylate) | 76.2 |
| | Monomer (Glyceryl propoxy triacrylate) | 19.8 |
| | Photoinitiator | 4 |
| **Composition C3** | Sodium sulfite lignosulfonate | 0.05 |
| | Cellulose derivative | 8 |
| | Distilled Water | 91.95 |

The percentage of leakage of the fragrance from capsules according to the invention were assessed and compared to microcapsules formulated with the method described in WO 2018210857 and WO 2018100179 without a viscosifying agent (Comp. 1 and Comp. 2).

The method of evaluation of leakage of the fragrance from capsules was performed in the following manner: A homogeneous slurry was prepared comprising the microcapsules and a liquid fabric softener base, as described in EP0459821B1.
Formulation of liquid fabric softener:

-    8% Tetranyl L690 Partially Hydrogenated Palm Esterquat
-    $CaCl_2.2H_2O$ (at 15%) 1.0%
-    Preservative 0.1%
-    Deionized Water Up to 100%

The slurry was left at rest for 24 hours and then rehomogenized immediately prior to centrifugation at 2000G for 10 minutes in a Multifuge X3Thermo Scientific, 42631189
4g of the resulting supernatant are mixed with 4 g of ethyl acetate, which is left at rest for 1 hour, after which 20 mg of NaCL are added under mixing. The resulting solution is centrifuged for 5 min at 2000G, and the supernatant is recovered for analysis by Gas Chromatography Flame ionization detection using a Trace 1300Thermo Fisher Scientific Detector, with a liquid injector Thermo Fisher Scientific AS1310.

The results are summarized in the below table:

| Composition | Fragrance | Core | Core Viscosity (mPa.s) | % Leakage |
|---|---|---|---|---|
| **Comparative (Comp. 1)** | Limonene | Liquid Core | 268 | 16±0.5 |
| **Invention (Inv. 1)** | Limonene | Viscous Core | 6402 | 11 ±0.5 |
| **Comparative (Comp. 2)** | Manzanate | Liquid Core | 647 | 18±0.2 |
| **Invention (Inv.2)** | Manzanate | Viscous Core | 6136 | 13±1 |

Example 1 shows that by changing the core from a liquid to a viscous core, the leakage from the capsule is reduced by at least 20%.

## Example 2 - **Preparation of microcapsules comprising a plasticizer**

The microcapsules according to the invention have been formulated by incorporating the viscosifying agent. The comparative example (Comp. 3) has been formulated without a viscosifying agent.

A mechanical stirrer (Ika Eurostar 20) equipped with a deflocculating stirring propeller is used to carry out all the stirring steps

### Step a): Creation of the Core of the Capsules (Dispersion of Particles - Composition C1)

Decanol 65%
Fumed silica 5%
Biodegradable carrier oil - complex ester oil (DEHYLUB® 4038 or Priolube 1847) 30%

14

The core of the capsule is obtained by mixing the above components at RT until homogeneous, thereby obtaining C1.

### Step b): Preparation of the shell composition

Shell composition:

- M1: aliphatic nonaromatic glycerol-modified polyester oligomer with acrylate terminal groups polyester comprising less than 3% of nitrogen by weight. 79%
- M2: tri-acrylated monomer. 20%
- Photoinitiator: Darocur 1173. 1%

The shell of the capsule is prepared by mixing the above components at RT until homogeneous, thereby obtaining C2.

The composition of the core C1 is added at RT to the composition of the shell C2 with a mixing rate of 100 rpm to produce the first emulsion E1. The ratio of the weight of the shell to the weight of the core is 40/60.

### Step c): Preparation of the Second Emulsion (E2)

Composition C3, the continuous phase, is produced from 8% sodium alginate, and 92% water. The composition C3 is stirred at 2,000 rpm until complete homogenization. The first emulsion E1 is then added to the composition C3, which is then stirred at 2,000 rpm for 2 minutes at RT to obtain the second emulsion E2, at a ratio of E1 :C3 of 10:90.

### Step d): Reticulation of the Capsule Envelope

The second emulsion (E2) obtained in the previous step is irradiated for 2 minutes with the aid of a UV light source (Dymax LightBox ECE 2000) having a maximum light intensity of 1 W/cm2 at a waveform length of 365 nm.

The microcapsules were separated from their continuous phase by centrifugation at 2000 G for over 3 cycles for a total of 20 min.

| | Raw materials | % in composition |
|---|---|---|
| **Composition C1** | Volatile Active (Decanol - Plastifying agent) | 63 |
| | Viscous Carrier oil (Chemical Name Esters - Complex ester, Priolube 1847 commercial name ) | 30 |
| | Viscosifying agent: fumed silica+methacrysilane | 7 |
| **Total** | | 100 |

| | Raw materials | % |
|---|---|---|
| **Composition C2** | aliphatic glycerol-modified polyester oligomer with acrylate terminal groups polyester comprising less than 3% of nitrogen by weight.) | 79 |
| | Propoxylated 3 Glyceryl Triacrylate | 20 |
| | Photoinitiator (Darocure) | 1 |
| **Composition C3** | Sodium sulfite lignosulfonate | 0.1 |
| | Cellulose derivative | 8 |
| | Distilled Water | 91.9 |

The determination of the %leakage after 7 days as shown in the table below was performed through Thermal Gravimetric Analysis, using a Texas Instruments TGA Q5000 Automatic Sample Processor. The slurry of microcapsules was placed in a silicon mold at ambient temperature. Measurements of the slurry were taken at T=0, T=3 days, T= 7 days and T=14 days at room temperature. 10g of the microcapsule slurry are used for each measurement. The sample is exposed to a temperature ramp 0 to 600°C. The boiling temperature of decanol is 230°C. The % weight loss at T=0 at this

EP 4 552 734 A1

temperature provides the baseline quantity of decanol in the microcapsule slurry.

The measured quantity of decanol at T=7 is below that of T=0, resulting from from leakage of decanol out of the capsule. The leakage as change in weight can be determined as (weight loss at T=7)/(weight loss at T)=0%. The weight % loss of the sample at T=7 was measured. The percentage of leakage of the fragrance from capsules according to the invention were assessed and compared to microcapsules formulated without rheological modifier (viscosifying agent). The results are summarized in the below table.

| Composition | Core | Viscosity (m$^2$/s) | % leakage after 7 days |
|---|---|---|---|
| Comp. 3 | Without rheological modifier | 59.51 | 70 |
| Inv. 4 | 7% fumed silica+methacrysilane | 10,442.92 | 50 |

The use of a rheological modifier reduced the leakage of decanol out of the capsule from 70% to 50%.

**Claims**

1. A biodegradable solid cross-linked microcapsule encapsulating at least one volatile ingredient or a preparation comprising a volatile ingredient, said microcapsule having a mean diameter from 0.5 µm to 100 µm, said microcapsule comprising:

    - a core consisting of a composition C1 comprising at least one volatile ingredient or a preparation comprising a volatile ingredient, and from 1% to 20% by weight of a viscosifying agent relative to the total weight of said composition, and
    - a solid cross-linked polymer envelope at least partially, preferably totally, encapsulating the core at its periphery, said solid cross-linked polymer envelope being made of a cross-linked polymer, said polymer being a polymer obtained by polymerizing one or several oligomer(s) or monomer(s),
    and wherein the average thickness of said solid cross-linked polymer envelope is from 5% to 30% of the average diameter of said solid cross-linked microcapsule.

2. The solid cross-linked microcapsule of claim 1, wherein the viscosifying agent is selected from the group consisting of: fumed silica, clay, organic polymers such as hydrogenated vegetable oils, polyesters, hydrogenated styrene/isoprene copolymer, polyamides, polyurethanes, oil-gelling polymers, hydrogels, such as polysaccharides or hydrophilic silicates, and mixtures thereof.

3. The solid cross-linked microcapsule of any one of claims 1 or 2, wherein the composition C1 further comprises from 5% to 80% by weight of a carrier oil relative to the total weight of said composition C1.

4. The solid cross-linked microcapsule of any one of claims 1 to 3, wherein the composition C1 comprises from 20% to 100%, preferably 30% to 80% by weight of volatile ingredient relative to the total weight of said composition.

5. The solid cross-linked microcapsule of any one of claims 1 to 4, wherein the solid cross-linked polymer envelope is made of a biodegradable cross-linked polymer, said polymer being an aliphatic polymer, obtained by polymerizing one or several oligomer(s) or monomer(s).

6. The solid cross-linked microcapsule of any one of claims 1 to 4, wherein the solid cross-linked polymer envelope is made of a biodegradable cross-linked polymer, said polymer being an aliphatic polymer obtained by polymerizing one or several oligomer(s) or monomer(s), said polymer comprising at least 20%, in particular at least 25%, and more preferably at least 30%, of ester groups by weight in comparison with the total weight of said polymer.

7. The solid cross-linked microcapsule of any one of claims 5 or 6, wherein the aliphatic polymer of the solid cross-linked polymer envelope is obtained by polymerizing one or several oligomer(s) or monomer(s) M1 and M2, the molecular weight of M1 being from 1,500 to 10,000 g/mol and the molecular weight of M2 being from 50 to 1,500 g/mol, said oligomers M1 and M2 comprising preferably at least one reactive function selected from the group formed by acrylate, methacrylate, vinyl ether, N-vinyl ether, epoxy, siloxane, amine, lactone, phosphate and carboxylate functions.

8. The solid cross-linked microcapsule of any one of claims 1 to 7, wherein the solid cross-linked polymer envelope is formed through UV polymerization.

9. The solid cross-linked microcapsule of any one of claims 1 to 8, being devoid of surfactant.

10. The solid cross-linked microcapsule of any one of claims 1 to 9, further comprising lignosulfonate.

11. A formulation comprising at least one solid cross-linked microcapsule according to any one of claims 1 to 10.

12. The use of the formulation of claim 11, in laundry care, cleaning products, home care, personal care, or hair care.

13. The use of the formulation of claim 11, in electronics, coatings, paints, construction materials, plastics, paper or textile functionalization.

Europäisches
Patentamt

European
Patent Office

Office européen
des brevets

# EUROPEAN SEARCH REPORT

Application Number

EP 23 30 6919

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2020/164332 A1 (DEMOULIN DAMIEN [FR] ET AL) 28 May 2020 (2020-05-28) * [0047], [0236], [0244]-[0245]; [0072], [0193] and [0236]-[0239]; [0056]; examples 1, 3 * | 1-13 | INV. B01J13/16 A61K8/11 A61Q5/00 A61Q19/00 A62D1/00 C11D3/50 |
| X | US 2022/119738 A1 (DEMOULIN DAMIEN [FR] ET AL) 21 April 2022 (2022-04-21) * [0019], [0031]-[0034] [0100]-[0114], [0188], [0275], [0314]; examples 1-3 * | 1-13 | |
| A | US 2022/106540 A1 (DEMOULIN DAMIEN [FR] ET AL) 7 April 2022 (2022-04-07) * [0176], [0263], [0302]-[0303], [0090]-[0104]; paragraph [0013] – paragraph [0025]; claims 1,4 * | 1-13 | |
| A | US 2019/388861 A1 (WALTERS JAMIE [FR] ET AL) 26 December 2019 (2019-12-26) * [0171], [0066]-[0067]; example 1 * | 1-13 | TECHNICAL FIELDS SEARCHED (IPC) |
| A | US 2020/038297 A1 (DEMOULIN DAMIEN [FR] ET AL) 6 February 2020 (2020-02-06) * [0150], [0157], [0268], [0308]-[0314], [0319]; examples 1-2 * | 1-13 | B01J A62D C11D A61Q A61K |

The present search report has been drawn up for all claims

| Place of search | Date of completion of the search | Examiner |
|---|---|---|
| The Hague | 18 April 2024 | Mc Donnell, Shane |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

# EP 4 552 734 A1

## ANNEX TO THE EUROPEAN SEARCH REPORT
## ON EUROPEAN PATENT APPLICATION NO.

EP 23 30 6919

This annex lists the patent family members relating to the patent documents cited in the above-mentioned European search report.
The members are as contained in the European Patent Office EDP file on
The European Patent Office is in no way liable for these particulars which are merely given for the purpose of information.

18-04-2024

| Patent document cited in search report | | | Publication date | Patent family member(s) | | | Publication date |
|---|---|---|---|---|---|---|---|
| US 2020164332 | A1 | | 28-05-2020 | CN | 110785224 | A | 11-02-2020 |
| | | | | EP | 3624932 | A1 | 25-03-2020 |
| | | | | FR | 3066116 | A1 | 16-11-2018 |
| | | | | JP | 2020520794 | A | 16-07-2020 |
| | | | | JP | 2023095871 | A | 06-07-2023 |
| | | | | KR | 20200044724 | A | 29-04-2020 |
| | | | | KR | 20240019382 | A | 14-02-2024 |
| | | | | US | 2020164332 | A1 | 28-05-2020 |
| | | | | WO | 2018210857 | A1 | 22-11-2018 |
| US 2022119738 | A1 | | 21-04-2022 | AU | 2020210829 | A1 | 12-08-2021 |
| | | | | CN | 113383063 | A | 10-09-2021 |
| | | | | EP | 3914683 | A1 | 01-12-2021 |
| | | | | FR | 3091877 | A1 | 24-07-2020 |
| | | | | JP | 2022518801 | A | 16-03-2022 |
| | | | | SG | 11202107953U | A | 30-08-2021 |
| | | | | US | 2022119738 | A1 | 21-04-2022 |
| | | | | WO | 2020152216 | A1 | 30-07-2020 |
| US 2022106540 | A1 | | 07-04-2022 | CN | 113383062 | A | 10-09-2021 |
| | | | | EP | 3914684 | A1 | 01-12-2021 |
| | | | | FR | 3091878 | A1 | 24-07-2020 |
| | | | | US | 2022106540 | A1 | 07-04-2022 |
| | | | | WO | 2020152222 | A1 | 30-07-2020 |
| US 2019388861 | A1 | | 26-12-2019 | AU | 2017367182 | A1 | 20-06-2019 |
| | | | | BR | 112019011248 | A2 | 15-10-2019 |
| | | | | CA | 3045215 | A1 | 07-06-2018 |
| | | | | CN | 110062779 | A | 26-07-2019 |
| | | | | EP | 3548529 | A1 | 09-10-2019 |
| | | | | ES | 2834874 | T3 | 21-06-2021 |
| | | | | FR | 3059666 | A1 | 08-06-2018 |
| | | | | JP | 7010965 | B2 | 10-02-2022 |
| | | | | JP | 2020500707 | A | 16-01-2020 |
| | | | | KR | 20190126048 | A | 08-11-2019 |
| | | | | US | 2019388861 | A1 | 26-12-2019 |
| | | | | US | 2020094214 | A1 | 26-03-2020 |
| | | | | WO | 2018100179 | A1 | 07-06-2018 |
| US 2020038297 | A1 | | 06-02-2020 | FR | 3064188 | A1 | 28-09-2018 |
| | | | | US | 2020038297 | A1 | 06-02-2020 |
| | | | | WO | 2018172430 | A2 | 27-09-2018 |

EPO FORM P0459

For more details about this annex : see Official Journal of the European Patent Office, No. 12/82

## REFERENCES CITED IN THE DESCRIPTION

**Patent documents cited in the description**

- CA 2774661 C
- WO 20180210857 A
- WO 2022117681 A
- WO 2018210857 A
- WO 2018100179 A
- EP 0459821 B1

**Non-patent literature cited in the description**

- **S. ARCTANDER**. Perfume and Flavor Chemicals. Montclair, 1969
- **S. ARCTANDER**. Perfume and Flavor Materials of Natural Origin. Elizabeth, 1960
- International Fragrance Association's list. IFRA
- Flavor and Fragrance Materials. Allured Publishing Co., 1991
- **BENÍTEZ, JOSE** ; **HEREDIA-GUERRERO, JOSÉ** ; **GUZMAN-PUYOL, SUSANA** ; **BARTHEL, MARKUS** ; **DOMINGUEZ, EVA** ; **HEREDIA, ANTONIO**. Polyhydroxyester Films Obtained by Non-Catalyzed Melt-Polycondensation of Natural Occurring Fatty Polyhydroxyacids.. *Frontiers in Materials*, 2015
- **G. LI BASSI**. Les photoinitiateurs dans la reticulation des revêtements'' (Photoinitiators in the crosslinking of coatings). *Double Liaison - Chimie des Peintures*, November 1985 (361), 34-41
- **HENRI STRUB**. Applications industrielles de la polymérisation photoinduite'' (Industrial applications of photoinduced polymerization). *L'Actualité Chimique*, February 2000, 5-13
- **MARC, J.M. ABADIE**. Photopolymères : considérations théoriques et réaction de prise'' (Photopolymers: theoretical considerations and curing reaction). *Double Liaison - Chimie des Peintures*, 1992 (435-436), 28-34